(19) Europäisches Patentamt
European Patent Office
Office européen des brevets

(11) Publication number: 0 278 686 A1

(12) # EUROPEAN PATENT APPLICATION

(21) Application number: 88300960.7

(22) Date of filing: 05.02.88

(51) Int. Cl.4: C07D 471/04, A61K 31/505, //(C07D471/04,239:00,221:00)

(30) Priority: 07.02.87 GB 8702802
07.02.87 GB 8702803

(43) Date of publication of application:
17.08.88 Bulletin 88/33

(84) Designated Contracting States:
AT BE CH DE ES FR GB GR IT LI LU NL SE

(71) Applicant: THE WELLCOME FOUNDATION LIMITED
183-193 Euston Road
London NW1 2BP(GB)

(72) Inventor: Rauckman, Barbara Seavey
Route 6 Box 165-AA Baptist Road
Durham North Carolina 27703(US)
Inventor: Woolley, Joseph Laird, Jr.
1509 Blount Street
Durham North Carolina 27707(US)
Inventor: Roth, Barbara
7 Lone Pine Drive
Chapel Hill North Carolina 27514(US)

(74) Representative: Rollins, Anthony John et al
Group Patents & Agreements The Wellcome Research Laboratories Langley Court
Beckenham Kent BR3 3BS(GB)

(54) **Pyridopyrimidines methods for their preparation and pharmaceutical formulations thereof.**

(57) The present invention relates to compounds of the general formula (III)

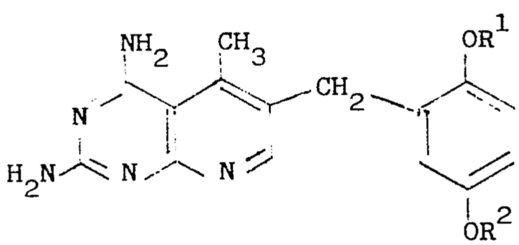

(III)

wherein $R^1$ is hydrogen when $R^2$ is methyl or $R^1$ is methyl when $R^2$ is hydrogen, and pharmaceutically acceptable salts thereof, i.e. the compounds 2,4-diamino-5-methyl-6-(2-hydroxy-5-methoxybenzyl)pyrido(2,3-d)pyrimidine and 2,4-diamino-5-methyl-6-(5-hydroxy-2-methoxybenzyl)pyrido(2,3-d)pyrimidine and their pharmaceutically acceptable salts.

The compounds have potent inhibitory activity against dihydrofolate reductase, and are useful in treating bacterial infections and in treating proliferative diseases, for example in mammals.

Compounds of formula (III) may be prepared for example by debenzylation of the corresponding benzyloxy derivative.

EP 0 278 686 A1

## PYRIDOPYRIMIDINES, METHODS FOR THEIR PREPARATION AND PHARMACEUTICAL FORMULATIONS THEREOF

The present invention relates to 2,4-diaminopyrido[2,3-d]pyrimidines, to pharmaceutical formulations comprising such compounds and to their use in medicine.

UK. Patent No. 1 084 103 (and corresponding U.S. Patent No. 3 322 765) discloses 2,4-diaminopyrido-[2,3-d]pyrimidines of the general formula (I):

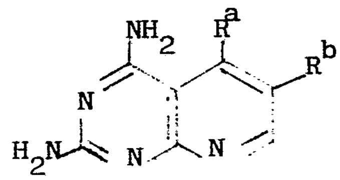

(I)

in which $R^a$ is hydrogen or alkyl and $R^b$ is inter alia an unsubstituted benzyl group or a benzyl group substituted by one or more halogen atoms, alkyl or alkoxy groups.

U.K. Patent No. 970 583 discloses compounds similar to those of formula (I) above but wherein $R^a$ is a hydrogen and $R^b$ is, inter alia, a phenalkyl group optionally bearing one or more substituents.

The compounds of formula (I) were described as having high in vitro and in vivo activity against bacteria or bacterial infections in experimental animals.

Subsequently it has been found that the compounds of formula (I) specifically disclosed in U.K. 1 084 103 show some inhibitory activity against mammalian dihydrofolate reductase (DHFR), and the activity was sufficient to render them potentially useful in the treatment of conditions where inhibition of mammalian DHFR is desirable.

It has further been found that many of these compounds are potent inhibitors of histamine N-methyltransferase (HMT), an enzyme involved in the metabolism of histamine. In this manner they often cause an undesirable accumulation of histamine in organs and tissues. The effects of histamine are well known and any possibility of a further utility for these compounds was substantially diminished by their strong inhibition of HMT.

Further investigation showed that a number of other compounds of formula (I) also possessed DHFR inhibitory activity but that these, too, were also potent inhibitors of HMT. Others, which had acceptably low levels of inhibition of HMT were found to have insufficient activity as inhibitors of DHFR.

European Patent Specification 0021292 and U.S. Patent No. 4372957 disclose that compounds of the formula (II):

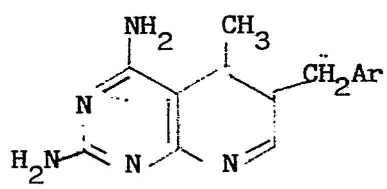

(II)

wherein Ar is

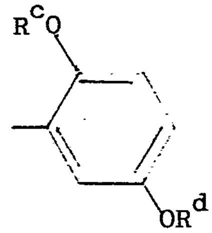

or

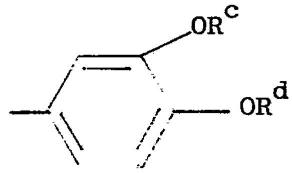

and $R^c$ and $R^d$ are lower ($C_{1-6}$) alkyl; and pharmaceutically acceptable acid addition salts thereof are not only very potent inhibitors of mammalian DHFR, but also have acceptably low inhibitory activity against

HMT, and are useful in the treatment of proliferative diseases, such as psoriasis, basal and squamous cell carcinomas of the skin, and various forms of cancer including leukemias, lymphomas, sarcomas and solid tumors. Preferably monobasic salts are provided.

2,4-Diamino-5-methyl-6-(2,5-dimethoxybenzyl)pyrido[2,3-d]pyrimidine is identified as a preferred compound.

The present invention concerns the desmethyl analogues of 2,4-diamino-5 -methyl-6-(2,5-dimethoxybenzyl)pyrido[2,3-d]pyrimidine, ie. 2,4-diamino-5 -methyl-6-(5-hydroxy-2-methoxybenzyl)pyrido[2,3-d]pyrimidine and 2,4-diamino-5-methyl-6-(2-hydroxy-5-methoxybenzyl)pyrido [2,3-d]pyrimidine which have potent inhibitory activity against dihydrofolate reductase, and which are useful in treating bacterial infections and in treating proliferative diseases, for example in mammals.

Accordingly, the present invention provides a compound of the general formula (III)

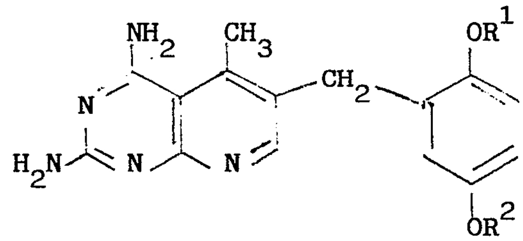

(III)

wherein $R^1$ is hydrogen when $R^2$ is methyl or $R^1$ is methyl when $R^2$ is hydrogen, and pharmaceutically acceptable salts thereof.

Thus the invention provides 2,4-diamino-5-methyl-6-(2-hydroxy)-5-methoxybenzyl)pyrido[2,3-d]pyrimidine and pharmaceutically acceptable salts thereof and 2,4-diamino-5-methyl-6-(5-hydroxy-2-methoxybenzyl)pyrido [2,3-d]pyrimidine and pharmaceutically acceptable salts thereof.

The biological activity of the compounds of the invention resides in the free base and thus the nature of the salt is of minor importance. Suitable acid addition salts include, for example, those derived from hydrochloric acid, hydroiodic acid, sulphuric acid, phosphoric acid, acetic acid, p-toluenesulphonic acid, methanesulphonic acid, maleic acid, lactic acid, citric acid, tartaric acid, succinic acid, oxalic acid, pchlorobenzenesulphonic acid, isethionic acid, glucuronic acid, pantothenic acid and lactobionic acid. Suitable basic salts include those formed with alkali metals such as sodium or potassium.

Compounds of formula (III) may be prepared by any method known in the art for the preparation of compounds of analogous structure.

Thus, for example compounds of formula (III) may be prepared by debenzylation of a corresponding benzyloxy compound of formula (IV):

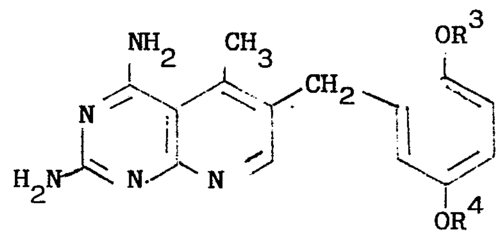

(IV)

wherein $R^3$ is benzyl when $R^4$ is methyl or $R^3$ is methyl when $R^4$ is benzyl or a salt thereof.

The debenzylation is carried out by any suitable method known to those skilled in the art, for example by catalytic hydrogenation i.e. with hydrogen in the presence of a transition metal catalyst such as palladium on carbon. This reaction will normally be carried out in an inert solvent, conveniently an alcohol such as the mono-methyl ether of ethylene glycol (2-methoxy ethanol) at a non-extreme temperature, for example between 0 and 100°C and conveniently at room temperature.

Compounds of formula (IV) may be prepared by methods well known to those skilled in the art. In particular, they may be prepared by the reductive cleavage of the corresponding 7-substituted compound of formula (V):

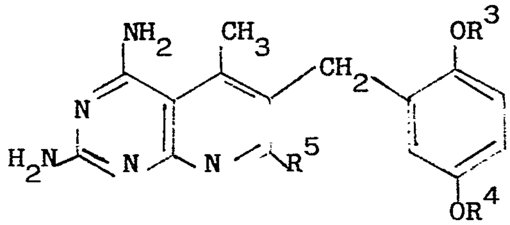

(V)

wherein $R^3$ and $R^4$ are as defined above and $R^5$ is a leaving group capable of being removed by hydrogenolysis. Such groups include for example a mercapto or a halogeno (e.g. chloro) group.

Where $R^5$ is SH the dethiation may for instance be conveniently effected by reaction with a desulphurizing agent such as Raney nickel or Raney cobalt or by catalytic hydrogenation utilizing hydrogen in the presence of a catalyst such as palladium on charcoal.

In the case where $R^5$ is a halogen atom the compounds of formula (IV) may for instance be conveniently obtained by, e.g., catalytic hydrogenation. Alternatively a halogen atom $R^5$ may be removed e.g. using zinc in aqueous ammonia.

It will be appreciated that when the group $R^5$ is removed by catalytic hydrogenation, the debenzylation may conveniently be effected simultaneously with cleavage of the $R^5$ group, giving a compound of formula (III) directly from a compound of formula (V). Thus, a further process for preparing compounds (III) comprises catalytic hydrogenation of a compound of formula (V) wherein $R^5$ is a group capable of being removed by catalytic hydrogenation.

Compounds of formula (V) wherein $R^5$ is a mercapto group may be prepared from the corresponding 7-chloro compound ($R^5$ = Cl) by reaction with a hydrosulfide as described in U.K. Patent No. 913,710 or by treatment of the corresponding 7-hydroxy compound with phosphorous pentasulfide.

Compounds of formula (V) wherein $R^5$ is halogen atom eg chlorine, may be prepared from the corresponding 7-oxo compound by halogenation eg. with a Vilsmeier reagent prepared from oxalyl or thionyl chloride. The 7-oxo compound may be prepared by reacting 2,4,6-triaminopyrimidine with a compound of formula (VI):

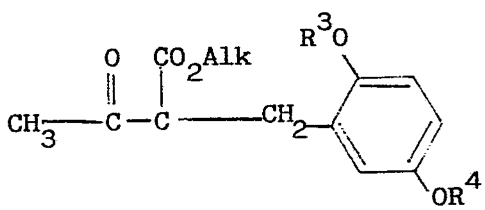

(VI)

wherein Alk is $C_{1-4}$ alkyl.

Compounds of formula (IV) may also be prepared directly by reacting 2,4,6-triaminopyrimidine with a compound of formula (VII)

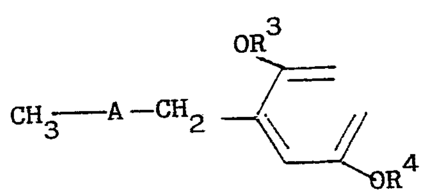

(VII)

wherein $R^3$ and $R^4$ are as defined above,

A is 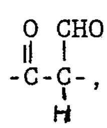, 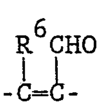 or 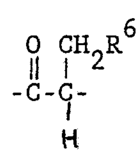

and $R^6$ is a leaving group such as for example a tertiary amino, alkoxy, alkythio, halogeno, sulphonate or tosylate group.

Compounds of formula (IV) may additionally be prepared by the conversion to amino groups, by methods known in themselves in pyrimidine chemistry, of the halogen atom(s), hydroxy and/or mercapto group(s) in a compound of formula (VIII)

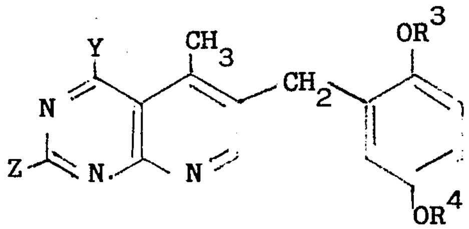

(VIII)

in which $R^3$ and $R^4$ are as defined above and Y and Z are the same or different and are halogen (e.g.Br,Cl) OH, SH or $NH_2$ provided that at least one of Y and Z is halogen, OH or SH. Reaction of this type are described in "Heterocyclic Compounds "Vol. 16, (Wiley, Interscience, 1962).

Compounds of formula (VIII) may be obtained by methods known in the art for preparing such compounds. In addition those in which Y is OH or $NH_2$ and Z is OH or SH may be obtained, for example, by reaction of urea, guanidine or thiourea with a suitable compound of formula (IX) :

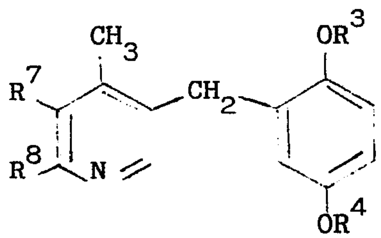

(IX)

in which $R^3$ and $R^4$ are as defined above, $R^7$ is $-CO_2H$,$-CO_2Alkyl$, - $CONH_2$ or CN and $R^8$ is $NH_2$, Cl or Br.

While it is possible for 2,4-diamino-5-methyl-6-(2-hydroxy-5-methoxybenzyl) pyrido[2,3-d]pyrimidine, or 2,4-diamino-5-methyl-6-(5-hydroxy-2-methoxybenzyl)pyrido[2,3-d]pyrimidine or a pharmaceutically acceptable salt thereof (hereinafter referred to as the "active compounds") to be administered as the raw chemical it is preferably presented in the form of a pharmaceutical formulation.

The invention therefore further provides a pharmaceutical formulation comprising the active compound together with a pharmaceutically acceptable carrier therefor. The carrier must be "acceptable" in the sense of being compatible with the other ingredients of the formulation and not deleterious to the recipient thereof.

The pharmaceutical formulations may be prepared by a method comprising bringing into association the active compound and a pharmaceutically acceptable carrier thereof.

Topical application is particularly suitable when the active compounds are for use in the treatment of proliferative skin diseases.

The term "topical" as applied herein relates to the use of the active ingredient incorporated in a suitable pharmaceutical carrier, and applied at the site of the disease for the exertion of local action.

Pharmaceutical formulations suitable for topical administration may be presented by anhydrous forms such as ointments, lotions, pastes, jellies, sprays, aerosols, and bath oils. The term ointment includes formulations (including creams) having oleaginous, absorption, water soluble and emulsion type bases, for example petrolatum, lanolin, polyethylene glycols and mixtures thereof.

Topical formulations may contain a concentration of the active ingredient of form 0.05 to 10% w/w preferably 0.1 to 2% w/w most preferably 0.2 to 1% w/w.

Other pharmaceutical formulations include those suitable for oral, rectal and parenteral administration, although of these oral is preferred. The formulations may, where appropriate, be conveniently presented in discrete dosage units and may be prepared by any of the methods well known in the art of pharmacy. A convenient unit dose formulation contains the active compound in an amount of from 1 mg to 1 g, preferably 2 mg to 500 mg, most preferably about 10 to 300 mg, to be taken once or several times daily.

All methods for the preparation of such formulations include the step of bringing into association the active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules, cachets or tablets each

containing a predetermined amount of the active compound. A tablet may be made by compression or moulding, optionally with one or more accessory ingredients. Compressed tablets may be prepared by compressing in a suitable machine the active compound in a free flowing form such as a powder or granules optionally mixed with a binder, lubricant, inert diluent, lubricating, surface active or dispersing agent. Moulded tablets may be made by moulding an inert liquid diluent. Tablets may be optionally coated and, if uncoated, may be optionally scored. Capsules may be prepared by filling the active compound either alone or in admixture with one or more accessory ingredients, into the capsule cases and then sealing them in the usual manner. Cachets are analogous to capsules wherein the active ingredient together with any accessory ingredient(s) is sealed in a rice paper envelope.

Pharmaceutical formulations suitable for oral administration wherein the carrier is a liquid may be presented as a solution or a suspension in an aqueous liquid, or as an oil-in-water or water-in-oil liquid emulsion.

Pharmaceutical formulations suitable for rectal administration wherein the carrier is a solid are most preferably presented as unit dose suppositories.

Suitable carriers include cocoa butter and other materials commonly used in the art, and the suppositories may be conveniently formed by admixture of the active compound with the softened or melted carrier(s) followed by chilling and shaping in moulds.

Pharmaceutical formulations suitable for parenteral administration include sterile solutions or suspensions of the active compound in aqueous or oleaginous vehicles. Such preparations are conveniently presented in unit dosage or multi-dose containers which are sealed after introduction of the formulation until required for use.

It should be understood that in addition to the aforementioned carrier ingredients the pharmaceutical formulations described above may include, as appropriate, one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

As has been described above the active compounds of the present invention are useful for the treatment of proliferative diseases. Thus they may be employed in a method for the treatment of a proliferative disease in a mammal, including man, which comprises the administration of an effective, amount of the active compound once or several times a day orally, or applied topically.

The compounds of the invention may also be employed in the manufacture of a medicament for the treatment of proliferative conditions.

The invention also provides the active compounds as hereinbefore defined or a pharmaceutically acceptable salt thereof for use in medicine.

The invention further provides the active compounds as hereinbefore defined or a pharmaceutically acceptable salt thereof for use in the treatment of proliferative conditions.

Proliferative conditions in which the active compounds of the invention may be employed include psoriasis, basal and squamous cell carcinomas of the skin and various forms of cancer, including leukemias, lymphomas, sarcomas and the solid tumours, and rheumatoid or proliferative arthritis.

The amount of active compound required for therapeutic effect as an antiproliferative agent will of course vary with such factors as the nature and severity of the disease, the age and weight of the patient, the route of administration and, where the compound is employed in salt form, the nature of the salt. In general a suitable dose for the treatment of a mammal (including man) will lie in the range of from 0.1 to 150 mg per kilogram bodyweight (mg/kg) per day, preferably in the range of 0.3 to 50 mg/kg, more preferably in the range of 0.5 to 20 mg/kg.

Toxic manifestations attributable to the active compound are typically those associated with folate depletion, such as bone marrow depression, megaloblastic changes, and gastrointestinal ulceration. Calcium leucovorin (calcium salt of 5-formyl-5,6,7,8-tetrahydrofolic acid) may be administered to effect reversal of these toxic manifestations or to prevent their occurrence. The administration of calcium leucovorin may be effected concurrently with treatment or at any state thereof whenever toxic symptoms appear.

Thus, the haematological activity of the active compound can be prevented or reduced by the simultaneous administration of leucovorin. Consequently, tissue levels of the active compound may be safely raised by increasing the dose of the compound together with a simultaneous administration of leucovorin.

The following Examples illustrate the invention and are not intended as a limitation thereof. In Examples 1 to 4 the active ingredient may be either 2,4-diamino-5-methyl-6-(2-hydroxy-5-methoxybenzyl)pyrido[2,3-d]pyrimidine or 2,4-diamino-5-methyl-6-(5-hydroxy-2-methoxybenzyl)pyrido[2,3-d] pyrimidine, or a salt thereof.

## EXAMPLE 1

### WATER SOLUBLE OINTMENT

| | |
|---|---|
| Active ingredient | 0.5 |
| Polyethylene glycol 300 | 20.0 |
| Polythylene glycol 1500 | 79.5 |
| Total | 100.0 |

## EXAMPLE 2

### SKIN CREAM

| | |
|---|---|
| Active ingredient | 0.5 |
| Glyceryl monosterate | 20.0 |
| Methylparaben | 0.3 |
| Petrolatum light liquid | 4.0 |
| Propylene glycol | 5.0 |
| Span 60 | 2.0 |
| Tween 61 | 4.0 |
| Water | 64.2 |
| Total | 100.0 |

"Span" and "Tween" are Trade Marks.

EXAMPLE 3

TABLET FORMULATION

| | |
|---|---|
| Active ingredient | 100 mg |
| Starch | 50 mg |
| Lactose | 50 mg |
| Stearic acid | 3 mg |
| Gelatin | 15 mg |
| Total | 218 mg |

EXAMPLE 4

INJECTABLE FORMULATION

| | |
|---|---|
| Active ingredient | 5 mg/ml |
| Propylene glycol | 40 ml |
| Ethanol | 11 ml |
| Water | 9 ml |

EXAMPLE 5

Synthesis of 2,4-diamino-5-methyl-6-(2-hydroxy-5-methoxybenzyl)pyrido [2,3-d]pyrimidine

A mixture of 2-benzyloxy-5-methoxybenzaldehyde (Helv. Chim. Acta, 1964, 47, 1996) (42.65 g), ethyl acetoacetate (26.5 g) and cyclohexane (300 ml), toluene (75 ml), piperidine (0.87 ml), and acetic acid (2.53 ml) was heated at reflux for 3 hours in an apparatus fitted with a Dean-Stark trap to collect the azeotropically distilled water. The solvent was removed, and then the intermediate was dissolved in EtOH (480 ml) and reduced on a Parr hydrogenator with 2,6 g 5% Pd/C under 20 psi of hydrogen for 3.5 hours. The product was purified on a silica gel column eluting with hexane: ethyl acetate, 9:1 giving 51 g (81%) of ethyl 2-(2-benzyloxy-5-methoxybenzyl)-3-oxobutyrate.
Anal. Calcd. for $C_{21}H_{24}O_5$:
C, 70:77; H, 6.79.
Found:
C, 70.63; H, 6.71

A mixture of the above product (21.81 g), 2,4,6-triaminopyrimidine (7.51 g), and 1-methyl-2-pyrrolidinone (50 ml) was heated for 2 hours at 190°C in an apparatus fitted with a Dean-Stark trap. Methanol (80 ml) was added to the cooled reaction mixture, and the resulting solid was collected by filtration and treated with boiling water to give 2,4-diamino-6-(2-benzyloxy-5-methoxybenzyl)-5-methylpyrido[2,3-d]-pyrimdin-7(8H)-one (9.79 g, 39%); mp 283-287°C.
Anal. Calcd. for $C_{23}H_{23}N_5O_3$:
C, 66.17; H, 5.55; N, 1678.
Found:
C, 66.30; H, 5.59; N, 16.53

This product was chlorinated as in Example 1 of U.S patent 4,372,957 (incorporated herein by reference) except that oxalyl chloride was used instead of thionyl chloride in the Vilsmeier reagent. The Vilsmeier reagent was prepared by slowly adding with stirring oxalyl chloride (13.4 ml) to a mixture of dimethylformamide (12.5 ml) and chloroform (80 ml) at 0°C and permitting to warm to, and stand at,

ambient temperature for 14 hours with stirring. The 2,4-diamino-6-(2-benzyloxy-5-methoxybenzyl)-5-methylpyrido[2,3-d]pyrimidine-7(8H)-one (6.74 g) was added to the Vilsmeier reagent, and the mixture was heated at reflux for 3 hours and then chilled to 0°C. After chlorination, n-butylamine (32 ml) was slowly added to the cooled reaction (below 30°C) followed by 1.5 hours of refluxing. The resulting solid was filtered, washed with chloroform, then EtOH, and heated in hot water to give 2.47 g (35%) of 2,4-diamino-6-(2-benzyloxy-5-methoxybenzyl)-7-chloro-5-methylpyrido[2,3-d]pyrimidine; mp 260-261°C.

Anal. Calcd. for $C_{23}H_{22}ClN_5O_2$:
C, 63.37; H, 5.09; N, 16.07; Cl, 8.13.
Found:
C, 63.10; H, 5.16; N, 15.96; Cl, 8.04

The chloro product from above (3.0 g - part was from a second batch) was refluxed in 2-methoxyethanol (350 ml) and dechlorinated and debenzylated by using sodium formate (2.82 g) and 5% Pd/C (1.4 g) added in portions over 4 hours. The product was purified on a silica gel column eluting with $CHCl_3$:MeOH/6:1 to give 0.34 g (18%) of 2,4-diamino-5-methyl-6-(2-hydroxy-5-methoxybenzylpyrido[2,3-d]-pyrimidine, mp 284-286°C.

Anal. Calcd. for $C_{16}H_{17}N_5O_2H_2O$:
C, 58.35; H, 5.81; N, 21.26.
Found:
C, 58.60; H, 5.60; N, 21.19

EXAMPLE 6

Synthesis of 2,4-diamino-5-methyl-6-(5-hydroxy-2-methoxybenzyl)pyrido[2,3-d]pyrimidine

A mixture of 5-benzyloxy-2-methoxybenzaldehyde (Chem. Ber. 1974, 107, 686) (10.1 g), ethyl acetoacetate (6.4 g) and cyclohexane (80 ml), toluene (20 ml), piperidine (0.2 ml), and acetic acid (0.6 ml) was heated at reflux for 3 hours in an apparatus fitted with a Dean-Stark trap to collect the azeotropically distilled water. The solvent was removed, and then the intermediate was dissolved in EtOH (110 ml) and reduced on a Parr hydrogenator with 0.5 g 5% Pd/C under 20 psi of hydrogen for 3.5 hours. The product was purified on a silica gel column eluting with hexane:ethyl acetate/9:1 giving 12.4 g (85%) of ethyl 2-(5-benzyloxy-2-methoxybenzyl)-3-oxobutyrate.

Anal. Calcd. for $C_{21}H_{24}O_5$:
C, 70.77; H, 6.79.
Found:
C, 70.88; H, 6.74

A mixture of the above product (12.0 g), 2,4,6-triaminopyrimidine (4.0 g), and 1-methyl-2-pyrrolidinone (30 ml) was heated for 2 hours at 190°C in an apparatus fitted with a Dean-Stark trap. Methanol (40 ml) was added to the cooled reaction mixture, and the resulting solid was collected by filtration and treated with boiling water to give 2,4-diamino-6-(5-benzyloxy-2-methoxybenzyl)-5-methylpyrido[2,3-d]pyrimidin-7(8H)-one (6.02 g, 45%); mp 338-342°C.

Anal. Calcd. for $C_{23}H_{23}N_5O_3$:
C, 66.17; H, 5.55; N, 16.78.
Found:
C, 66.08; H, 5.59; N, 16.70

This product was chlorinated as in Example 1 of U.S. patent 4,372,957 except that oxalyl chloride was used instead of thionyl chloride in the Vilsmeier reagent. The Vilsmeier reagent was prepared by slowly adding with stirring oxalyl chloride (12.1 ml) to a mixture of dimethylformamide (10.8 g) and chloroform (70 ml) at 0°C and permitting to warm to, and stand at, ambient temperature for 14 hours with stirring. The 2,4-diamino-6-(5-benzyloxy-2-methoxybenzyl)-5-methylpyrido[2,3-d]pyrimidin-7(8H)-one (5.72 g) was added to the Vilsmeier reagent, and the mixture was heated at reflux for 3 hours and then chilled to 0°C. After chorination, n-butylamine (29 ml) was slowly added to the cooled reaction mixture followed by 1.5 hours of refluxing. The resulting solid was filtered, washed with EtOH, and heated in hot water to give 4.92 g (82%) of 2,4-diamino-6-(5-benzyloxy-2-methoxybenzyl)-7-chloro-5-methylpyrido[2,3-d]pyrimidine; mp 249-251°C.

Anal. Calcd. for $C_{23}H_{22}ClN_5O_2.H_2O$:
C, 60.86; H, 5.33; N, 15.43; Cl, 7.81.
Found:
C, 60.90; H, 5.31; N, 15.43; Cl, 7.80

The chloro product from above (1.09 g) was refluxed in 2-methoxyethanol (200 ml) and dechlorinated and debenzylated by using sodium formate (1.02 g, 6 equiv.) and 5% Pd/C (0.8 g) added in portions over 4 hours. The product was purified on a silica gel column eluting with $CHCl_3$:MeOH/3:1 to give 0.62 g (83%) of 2.4-diamino-5-methyl-6-(5-hydroxy-2-methoxybenzyl) pyrido[2,3-d]pyrimidine, mp 266-270°C.

Anal. Calcd. for $C_{16}H_{17}N_5O_2.1.2\ H_2O$:
C, 57.72; H, 5.87; N, 21.03
Found:
C, 57.72; H, 5.51; N, 20.97.

EXAMPLE 7

Biological Activity

Test Compounds

(A) 2,4-diamino-5-methyl-6-(2-hydroxy-5-methoxybenzyl)pyrido[2,3-d] pyrimidine
(B) 2,4-diamino-5-methyl-6-(5-hydroxy-2-methoxybenzyl)pyrido[2,3-d] pyrimidine

A. In vitro DHFR Binding Affinities

The inhibition by the title compound of DHFR isolated from bacterial and mammalian sources was determined using known procedures [B. Roth et al., J. Med. Chem., 24, 933 (1981)].

| | DHFR Source | |
|---|---|---|
| | E. Coli | Rat liver |
| | $I_{50}$ (nM) | $I_{50}$ (nM) |
| Compound A | 8 | 18 |
| Compound B | 16 | 12 |

B. In vitro Cytotoxicity

The cytotoxicity of the test compounds was determined in cell culture using the human T-cell line CCRF/CEM (ATCC CCL 119) and also the human breast cancer cell line MCF-7 [H.D. Soule, et al., J.. Nat. Cancer Inst., 51, 1409 (1973)].

| | Cell line | |
|---|---|---|
| | CCRF/CEM | MCF-7 |
| | $ED_{50}$ (μM) | $ED_{50}$ (μM) |
| Compound A | 0.40 | 0.79 |
| Compound B | 0.08 | 0.75 |

EXAMPLE 8

Synthesis of 2,4-diamino-5-methyl-6-(2-hydroxy-5-methoxybenzyl)pyrido -[2,3-d]pyrimidine

Ammonia was bubbled into a heated (90°C. bath temperature) mixture of 2,4-diamino-6-(2-benzyloxy-5-methoxybenzyl)-7-chloro-5-methylpyrido(2,3-d)-pyrimidine (30g) and zinc powder (30g) in methyl cellosolve (200 mL) and water (200mL) with stirring for 22 hr. The reaction mixture was filtered hot and the residue washed with methyl cellosolve (200mL). The combined filtrates were concentrated under reduced pressure and chromatographed on a silica gel (1.5 kg) column, eluting with 0-15% methanol in chloroform. Evaporation of the fractions containing the desired product yielded 2,4-diamino-6-(2-benzyloxy-5-methoxybenzyl)-5-methylpyrido [2,3-d]pyrimidine (9.1g) as an off-white solid, m.p. 228-230°C.(dec.).

Anal. Calcd. for $C_{23}H_{23}N_3O_2$, 0.07$CHCl_3$, 0.5.$CH_3OH$:
C,66.84; H, 5.93; N, 16.45; Cl, 1.75.
Found:
C,66.35; H, 5.93; N, 16.44; Cl, 1.59.

A mixture of 2,4-diamino-6-)2-benzyloxy-5-methoxybenzyl)-5-methylpyrido-[2,3-d]pyrimidine (3.0 g), 5% Pd/C-50% $H_2O$ (2.0 g) and sodium formate (8.0 g) in methyl cellosolve (200mL) was heated (90°C. bath temperature) with stirring for 18 hr. The reaction mixture was filtered, concentrated under reduced pressure and chromatographed twice on a silica gel column, eluting with 0.30% methanol in chloroform. Evaporation of the fraction containing the desired product yielded 2,4-diamino-5-methyl-6-(2-hydroxy-5-methoxybenzyl)-pyrido[2,3,-d]pyrimidine (0.30 g) which was identical with the product of Example 5.

## Claims

1. A compound of formula (III)

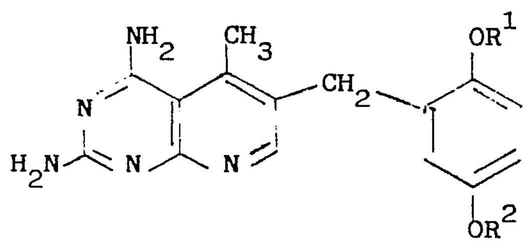

(III)

wherein $R^1$ is hydrogen when $R^2$ is methyl or $R^1$ is methyl when $R^2$ is hydrogen and pharmaceutically acceptable salts thereof.

2. 2,4-diamino-5-methyl-6-(2-hydroxy-5-methoxybenzyl)pyrido[2,3-]pyrimidine and pharmaceutically acceptable salts thereof.

3. 2,4-diamino-5-methyl-6-(5-hydroxy-2-methoxybenzyl)pyrido[2,3]pyrimidine and pharmaceutically acceptable salts thereof.

4. A pharmaceutical formulations comprising a compound of formula (III) together with a pharmaceutically acceptable carrier therefor.

5. A process for preparing a compound of formula (III) which comprises

a) debenzylation of a compound of formula (IV):

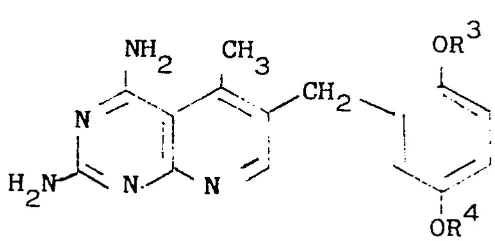

(IV)

wherein $R^3$ is benzyl when $R^4$ is methyl or $R^3$ is methyl when $R^4$ is benzyl, or a salt thereof, or

b) catalytic hydrogenation of a compound of formula (V)

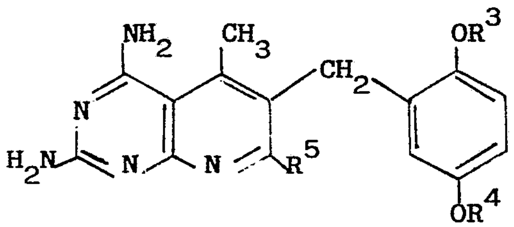

(V)

wherein $R^3$ and $R^4$ are as defined above and $R^5$ is a leaving group capable of being removed by catalytic hydrogenation.

6. A method of treatment of a proliferative disease in a mammal which comprises the administration of an effective amount of a compound of formula (III) or a salt thereof.

7. The use of a compound of formula (III) or a salt thereof in the manufacture of a medicament for the treatment of proliferative conditions.

8. A compound of formula (III) or a salt thereof for use in the treatment of proliferative conditions.

Claims for the following contracting States: SP, GR

1. A process for preparing a compound of formula (III)

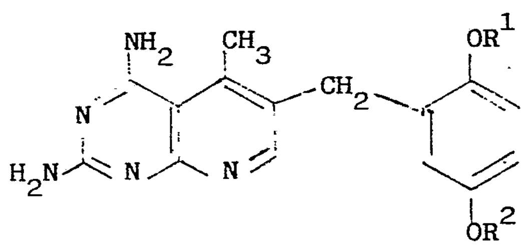

(III)

wherein $R^1$ is hydrogen when $R^2$ is methyl or $R^1$ is methyl when $R^2$ is hydrogen and pharmaceutically acceptable salts thereof, which process comprises

a) debenzylation of a compound of formula (IV):

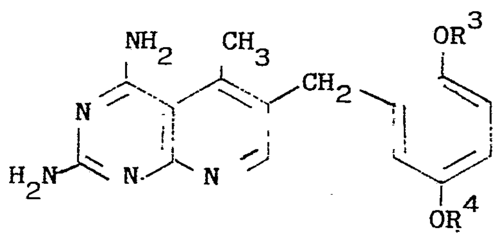

(IV)

wherein $R^3$ is benzyl when $R^4$ is methyl or $R^3$ is methyl when $R^4$ is benzyl, or a salt thereof, or

b) catalytic hydrogenation of a compound of formula (V)

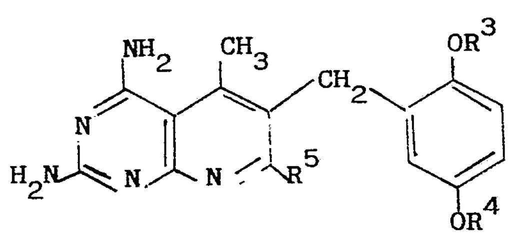

(V)

wherein $R^3$ and $R^4$ are as defined above and $R^5$ is a leaving group capable of being removed by catalytic hydrogenation.

2. A process according to claim 1 for preparing 2,4-diamino-5-methyl-6-(2-hydroxy-5-methoxybenzyl)-pyrido[2,3-]-pyrimidine and pharmaceutically acceptable salts thereof.

3. A process according to claim 1 for preparing 2,4-diamino-5-methyl -6-(5-hydroxy-2-methoxybenzyl)-pyrido[2,3]-pyrimidine and pharmaceutically acceptable salts thereof.

4. A process according to any of claims 1 to 3 wherein step (a) is effected by catalytic hydrogenation.

5. A process according to any of claims 1-4 wherein catalytic hydrogenation is effected in the presence of an inert solvent.

6. A process for preparing a pharmaceutical formulation which comprises bringing into association a compound of formula (III) and a pharmaceutically acceptable carrier therefor.

7. A method of treatment of a proliferative disease in mammal which comprises the administration of an effective amount of a compound of formula (III) or a salt thereof.

8. The use of a compound of formula (III) or a salt thereof in the manufacture of a medicament for the treatment of proliferative conditions.

9. A compound of formula (III) or a salt thereof for use in the treatment of proliferative conditions.

European Patent Office

# PARTIAL EUROPEAN SEARCH REPORT

which under Rule 45 of the European Patent Convention shall be considered, for the purposes of subsequent proceedings, as the European search report

Application number

EP 88300960.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | GB - A - 1 084 103 (THE WELLCOME FOUNDATION)<br>* Page 1, lines 9-29; page 2, lines 4-23 *<br>-- | 1-4 | C 07 D 471/04<br>A 61 K 31/505<br>//(C 07 D 471/04,<br>C 07 D 239:00,<br>C 07 D 221:00) |
| A | GB - A - 1 088 102 (THE WELLCOME FOUNDATION)<br>* Page 1, lines 14-18; page 2, lines 1-8,23,24 *<br>-- | 1-5 | |
| D,A | GB - A - 970 583 (THE WELLCOME FOUNDATION)<br>* Page 1, lines 19-28; page 2, lines 16-35 *<br>-- | 1-4 | |
| D,A | GB - A - 913 710 (THE WELLCOME FOUNDATION)<br>* Page 1, lines 35-60 *<br>-- | 1-5 | TECHNICAL FIELDS SEARCHED (Int. Cl.4)<br>C 07 D 471/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5,7,8

Claims searched incompletely: -

Claims not searched: 6

Reason for the limitation of the search: Article 52(4) EPC; method for treatment of human or animal body by therapy

| Place of search | Date of completion of the search | Examiner |
|---|---|---|
| VIENNA | 29-03-1988 | ONDER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

# PARTIAL EUROPEAN SEARCH REPORT

Application number

-2-

EP 88300960.7

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| D,A | EP - A1 - 0 021 292 (THE WELLCOME FOUNDATION)<br>* Claims 1,4,10,11 *<br>---- | 1-5,7, 8 | |
| | | | TECHNICAL FIELDS SEARCHED (Int. Cl.4) |

EPO Form 1505.3 06.78